# EUROPEAN PATENT APPLICATION

(11) **EP 4 094 728 A1**
(43) Date of publication of application: **30.11.2022**
(21) Application number: 21744603.8
(22) Date of filing: 12.01.2021
(51) Int. Cl.: A61F 2/38

(54) **TIBIAL COMPONENT**

(30) Priority: 21.01.2020 JP 2020007498
(71) Applicant: Kyocera Corporation, Kyoto-shi Kyoto 612-8501 (JP)
(72) Inventor: SUGIMOTO, Kazutaka, Kyoto-shi, Kyoto 612-8501 (JP)
(74) Representative: TBK
(86) International application number: PCT/JP2021/000619
(87) International publication number: WO 2021/149529

(57) **Abstract**

In a tibial component, a tibial block is fixed to a keel portion of a tibial tray. Accordingly, an augment member can be easily attached to the tibial tray without a through hole being formed in the tibial tray.

## Description

### Technical Field

The present invention relates to a tibial component of a knee prosthesis.

### Background Art

A tibial component for constructing a knee prosthesis is composed of a tibial plate in contact with a femoral component, a tibial tray having an upper surface for attaching the tibial plate, and the like.

In tibial components for revision operations, an augment member such as a tibial block or tibial wedge is attached to the lower surface of the tibial tray, that is, the surface opposite to the upper surface.

Known tibial trays are formed with a through hole extending from the lower surface to the upper surface in order to attach the augment member.

### Summary

### Technical Problem

In a case where the above-mentioned through hole is formed in the tibial tray, when the contact surface of the tibial plate and the upper surface of the tibial tray slide slightly, the edge of the through hole may scrape on the contact surface of the tibial plate (backside wear).

Known tibial plates are also known to include configurations for attaching the augment member without forming the through hole.

One aspect of the present invention realizes a tibial component to which an augment member can be attached by a simple procedure without requiring a through hole from the lower surface to the upper surface of the tibial tray.

### Solution to Problem

The tibial component according to one aspect of the present invention is a tibial component that forms a knee prosthesis, the tibial component including a tray having a main surface, a columnar portion disposed on the main surface, and a protruding portion protruding outward from a side surface of the columnar portion; an augment member disposed on the main surface; and a fixing mechanism configured to fix the augment member to the protruding portion.

### Advantageous Effects of Invention

According to this aspect of the present invention, there is no need for a through hole from a lower surface to an upper surface of the tibial tray, and so a tibial component that allows an augment member to be attached with a simple procedure is realized.

### Brief Description of Drawings

FIG. 1 is a schematic view illustrating an example of a configuration of a knee prosthesis according to an aspect of the present invention.
FIG. 2 is a perspective view of a tibial component.
FIG. 3 is a plan view of a tibial tray as viewed from below.
FIG. 4 is a posterior view of the tibial tray.
FIG. 5 is a cross-sectional view of the tibial tray taken along line A-A in FIG. 4.
FIG. 6 is a plan view of the tibial block viewed from below.
FIG. 7 is a plan view of the tibial component viewed from below.
FIG. 8 is a cross-sectional view of the tibial component taken along line B-B in FIG. 7.
FIG. 9 is a perspective view of the tibial component.
FIG. 10 is a plan view of the tibial tray viewed from below.
FIG. 11 is a posterior view of the tibial tray.
FIG. 12 is a perspective view of the tibial block.
FIG. 13 is a cross-sectional view of the tibial block taken along a plane parallel to the upper surface.
FIG. 14 is a schematic view illustrating a fixing pin.
FIG. 15 is a perspective view of the tibial component.
FIG. 16 is a cross-sectional view of the tibial component taken along line C-C in FIG. 15.

### Description of Embodiments

### First Embodiment

The following describes in detail a first embodiment of the present invention.

The knee is a joint formed by three bones, the femur F, the tibia T, and the patella. For a patient with a highly impaired knee joint, a knee prosthesis replacement procedure may be applied to re-acquire the original function of the patient's knee joint. In a knee prosthesis replacement procedure, the bone and cartilage of the damaged knee joint are resected, and replaced with an artificial replacement (implant) made of a metal or plastic with bioaffinity. This artificial replacement is called a "knee prosthesis".

### Configuration of Knee Prosthesis 100

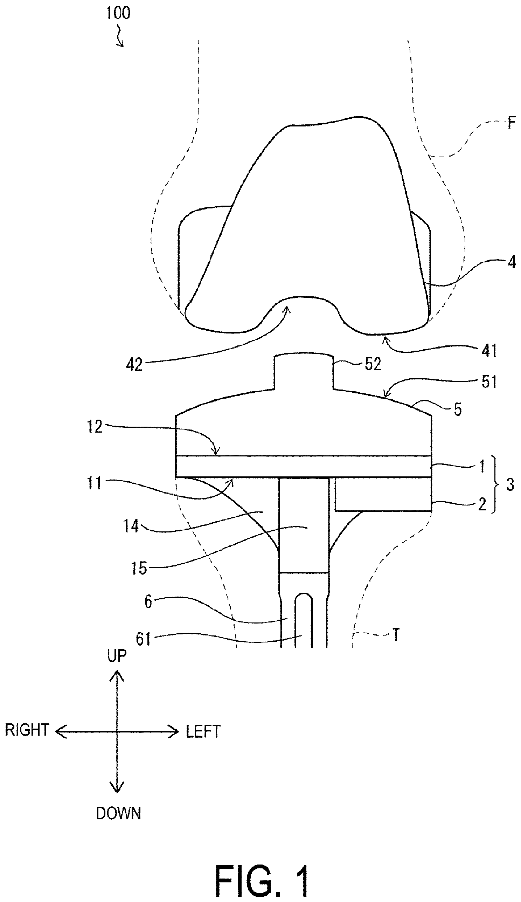

The configuration of a knee prosthesis 100 will now be described using FIG. 1. FIG. 1 is a schematic view illustrating an example of a configuration of a knee prosthesis 100 according to an aspect of the present invention. Note that in FIG. 1, contour images of the femur F and the tibia T are illustrated as dashed lines when the knee prosthesis 100 is placed in the knee joint of the patient. FIG. 1 shows the knee joint when the patient's knee is viewed from the anterior. Note that in FIGS. 1 to 16, the direction towards the patient's waist is defined as "up", the direction towards the patient's foot is defined as "down", the direction towards the patient's right leg is defined as "right" and the direction towards the patient's left leg is defined as "left".

### Femoral Component 4

A femoral component 4 is used to repair or replace the patient's femur F. The femoral component 4 is designed to articulate with a tibial component 3 or a tibial plate 5. Note that the femoral component 4 may be designed to articulate with a patella component (not illustrated) if desired. The femoral component 4 is attached to a distal end of the femur F. The femoral component 4 is formed with a metallic material with bioaffinity (such as cobalt-chromium-molybdenum alloy) or a ceramic material (such as zirconia ceramic).

When placed in the patient's femur F, the femoral component 4 has a U shape or a J shape as viewed from the side. The femoral component 4 covers the anterior and posterior sides of the femur F as well as an end portion of the femur F. The femoral component 4 is formed with a sliding portion 41 that is in contact with the tibial plate 5 described below, and a guide groove 42 provided in the anterior-posterior direction.

### Tibial Plate 5

The tibial plate 5 is used in combination with the tibial component 3 described below to repair or replace the tibial plateau of the knee joint. The tibial plate 5 is designed to articulate with the femoral component 4. The tibial plate 5 may be fixed by fitting with a tibial tray 1 described below. The tibial plate 5 may be formed using a plastic material (such as polyethylene) having bioaffinity and abrasion resistance. The tibial plate 5 is also referred to as a "tibial insert". The tibial plate 5 has a sliding surface 51 on which slides the femoral component 4 and a post portion 52.

The post portion 52 protrudes upward and is inserted into the guide groove 42 of the femoral component 4. In the tibial plate 5, when the patient flexes their knee, the post portion 52 moves back and forth along guide groove 42.

### Tibial Component 3

The tibial component 3 is used to repair or replace the patient's tibia T. The tibial component 3 is designed to articulate with the femoral component 4. The tibial component 3 is attached to the proximal end of the tibia T (such as to the tibial plateau). The tibial component 3 includes at least the tibial tray 1 (tray) and the tibial block 2 (augment member).

The tibial tray 1 has an upper surface 12 that is in contact with the tibial plate 5 and a lower surface 11 (main surface) that is a surface on the opposite side to the upper surface 12, which is a surface on the side in contact with the patient's tibia T. The lower surface 11 of the tibial tray 1 may have a stem portion 15 (columnar portion) that is columnar or tubular and that protrudes from the lower surface 11, and a keel portion 14 (protruding portion) that protrudes outward from a side surface of the stem portion 15.

The stem portion 15 has a mechanism for connecting an extension stem 6, which is described below, to the tibial tray 1. For example, when the stem portion 15 is a tubular member, a female thread into which a male thread provided in the extension stem 6 is screwed can be formed on the inner wall thereof.

On the other hand, the keel portion 14 is a protruding portion that is provided so that the stem portion 15 inserted into the medullary cavity of the tibia T does not rotate in the medullary cavity. In the present specification, a tibial tray 1 having two keel portions 14 is described as an example. The number of keel portions 14 may be freely selected and is not limited to being two.

The tibial component 3 according to one aspect of the present invention employs a fixing mechanism that fixes the tibial block 2 to the keel portion 14 disposed on the lower surface 11 of the tibial tray 1.

The tibial block 2 is an augment member that is used attached to the lower surface 11 of the tibial tray 1. The tibial block 2 is optionally used to increase the support of the tibial component 3 by compensating for bone loss between the lower surface 11 and the patient's tibia T. The tibial block 2 may be disposed from the center to the right side of the lower surface 11, or from the center to the left side of the lower surface 11. The tibial block 2 may be disposed at least partially to the right side of the center of the bottom surface 11 or at least partially to the left side of the center of the bottom surface.

The present disclosure describes a tibial block 2 mainly covering a portion (approximately half) of the lower surface 11. However, the present disclosure is not limited to this arrangement, and the tibial block 2 may be sized to cover at least half, or nearly all, of the lower surface 11.

Note that a tibial wedge (augment member) is well-known as an augment member used for the same purpose as the tibial block 2. The tibial block 2 has a semi-circular plate shape with a substantially uniform thickness, whereas the tibial wedge has a wedge shape in which one end is thicker than the other. In other words, whereas the upper surface 22 (first surface) and the lower surface 21 (second surface) of the tibial block 2 are substantially parallel, the upper and lower surfaces of the tibial wedge are not parallel. When fitted to the patient's tibia the thickness of the tibial wedge may, for example, become thinner going from right to left, or going left to right. Alternatively, when installed in the tibia, it may become thinner going from the posterior side toward the anterior side. In the present specification, the tibial component 3 to which the tibial block 2 is applied as an augment member, is described as an example, but the configuration is not limited to this. For example, in the tibial component 3, a tibial wedge may be applied as the augment member in place of the tibial block 2. Also, the tibial wedge may be sized to cover at least half, or nearly all, of the lower surface 11.

The extension stem 6 is an optional member used to increase the support of the tibial component. The extension stem 6 has a rod shape and is inserted into the medullary cavity of the tibia T. Note that a protruding portion 61 protruding outwardly from the surface of the extension stem may be formed so that the extension stem 6 does not rotate in the medullary cavity. Note that the extension stem 6 is not an essential configuration for the tibial component 3 according to one aspect of the present invention. Hence, in FIG. 1, only a proximal portion of the extension stem 6 is depicted, and a distal portion is omitted.

### Configuration of Tibial Component 3

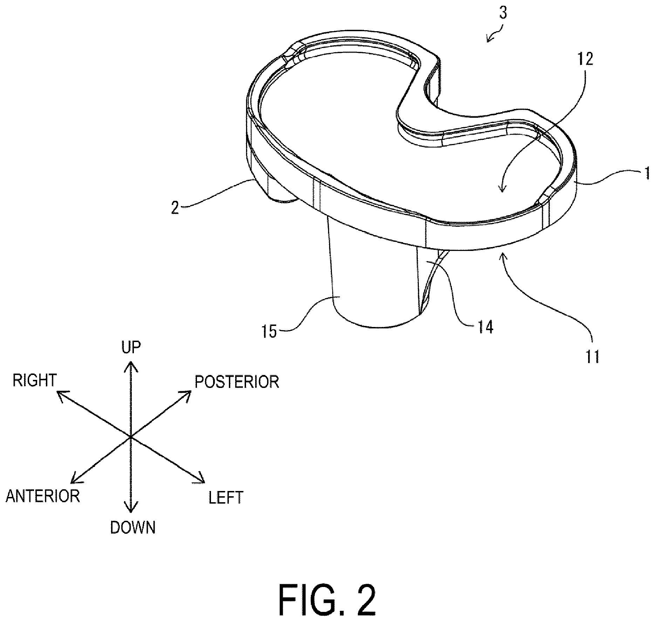

Next, the configuration of the tibial component 3 will be described with reference to FIG. 2 FIG. 2 is a perspective view of the tibial component 3. Note that the tibial component 3 illustrated in FIG. 2 has a tibial block 2 disposed in the tibial tray 1.

As illustrated in FIG. 2, the tibial tray 1 does not have a through hole formed from the lower surface 11 to the upper surface 12. Further, there is no recessed portion formed in the upper surface 12 of the tibial tray 1. As a result of configuring the tibial tray 1 in this way, even if the contact surface of the tibial plate 5 and the upper surface 12 of the tibial tray 1 slide, the contact surface of the tibial plate 5 is not scraped. Note that the outer edge of the upper surface of the tibial tray 1 may be formed with an edge portion 121 that mates with the tibial plate 5.

### Configuration of Tibial Tray 1

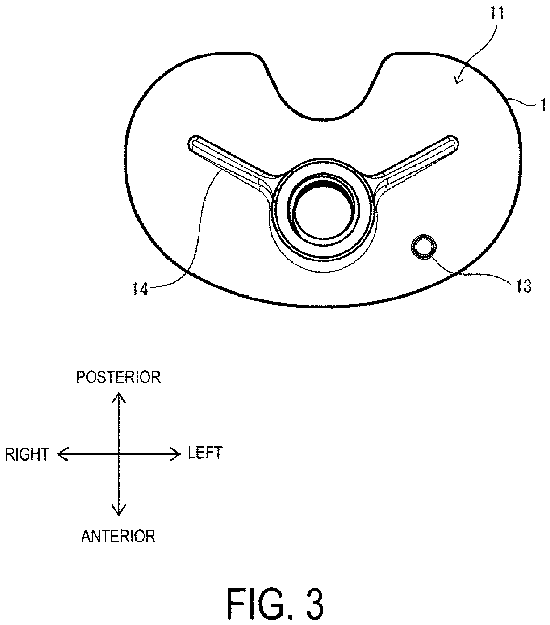

The configuration of the tibial tray 1 will now be described using FIG. 3.

FIG. 3 is a plan view of the tibial tray 1 viewed from below. As illustrated in FIG. 3, the keel portion 14 is formed to protrude radially from the side surface of the stem portion 15 and extends outward from the stem portion 15. The keel portion 14 is formed such that the tibial block 2 is not obstructed when it is attached at the attachment position on the lower surface 11 of the tibial tray 1. In FIG. 3, a tibial tray 1 having the keel portion 14 formed in two locations is illustrated. Note that the number of the keel portions 14 is not limited to two, and may be set as desired. Note that the number of keel portions 14 may be one or three or more. Also, the keel portions 14 can be provided in desired positions.

Additionally, the protruding form of the keel portion 14 does not need to be uniform. For example, the tibial tray 1 may be configured with a keel portion 14 that protrudes a large amount from the side surface of the stem portion 15 and a keel portion 14 that protrudes a small amount from the side surface of the stem portion 15.

The lower surface 11 of the tibial tray 1 may have a recessed portion 13 that is non-penetrating, although this is a non-essential configuration. The recessed portion 13 is disposed so as to face a through hole 24, described later, which is provided in the tibial block 2.

### Keel Portion 14

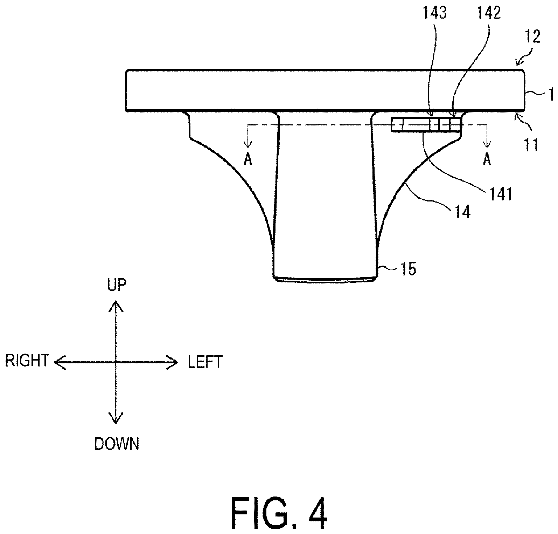

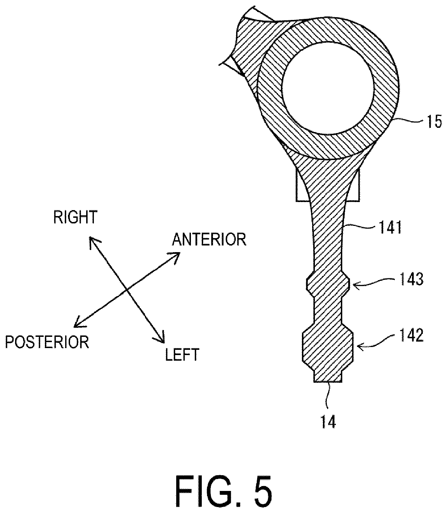

Next, the configuration of the keel portion 14 will be described in detail with reference to FIGS. 4 and 5. FIG. 4 is a view of the tibial tray 1 as viewed from the posterior side. FIG. 5 is a cross-sectional view of the tibial tray 1 taken along line A-A in FIG. 4.

FIG. 4 illustrates, as an example, a tibial tray 1 which allows the tibial block 2 to be attached to the left side of the lower surface 11. As illustrated in FIG. 4, at least one groove 141 (sliding groove) is formed on the keel portion 14 on the side of the attachment position of the tibial block 2.

The groove 141 is included in a fixing mechanism that secures the tibial block 2 to the keel portion 14 along with a contact portion 23 that is disposed in the tibial block 2. The groove 141 has a bottom surface where the keel portion 14 and the contact portion 23, which comes into contact with and sandwiches the keel portion 14, are in contact with each other. Note that the contact portion 23 of the tibial block 2 will be described later with specific examples.

As illustrated in FIG. 5, a projecting portion 142 and a projecting portion 143 may be formed on the bottom surface of the groove 141. In the projecting portion 142 and the projecting portion 143, the bottom surface of the groove 141 protrudes in the thickness direction of the keel portion 14 relative to the periphery. In other words the projecting portion 142 and the projecting portion 143 are "ridges" (or "bumps") in the groove 141.

When the tibial block 2 moves between the attachment position and the release position that is offset from the attachment position, the contact portion 23 slides on the bottom surface of the groove 141. The friction between the contact portion 23 and the groove 141 increases at positions where the projecting portion 142 and the projecting portion 143 are disposed. By arranging the projecting portion 142 and the projecting portion 143 at appropriate positions in the groove 141, the tibial block 2 can be fixed so that it is not easily removed from the keel portion 14.

Note that, if the tibial block 2 can be fixed so it is not easily detached from the keel portion 14, the projecting portion 142 and the projecting portion 143 are not required in the groove 141. For example, a friction surface that suppresses sliding of the contact portion 23 may be formed on at least a portion of the bottom portion of the groove 141. For example, a rough surface may be provided on at least a portion of the bottom portion of the groove 141 that is in contact with the contact portion 23. With this configuration, the sliding of the contact portion 23 is suppressed at any position where the rough surface is provided at the bottom portion of the groove 141.

It is to be noted that the position of the bone defect in the tibia T will be different for each patient. Hence, the position to be compensated for by the tibial block 2 may be on the right or left side of the tibial tray 1. In some cases, the bone defect may be large, and a prosthesis may thus be necessary all the way across the lower surface 11 of the tibial tray 1, from the right side to the left side, or even for the whole of the lower surface 11. Thus, the groove 141 may also be formed in the keel portion 14 on the left side in the tibial tray 1 illustrated in FIG. 4. In this case, the aforementioned recessed portion 13 may be formed at left-right symmetric positions on the lower surface 11 of the tibial tray 1. As another embodiment of the tibial block 2, the tibial block 2 may have a size so as to cover at least half of the lower surface 11, or may have a size that is substantially equal to the lower surface 11 in plan view.

### Configuration of Tibial Block 2

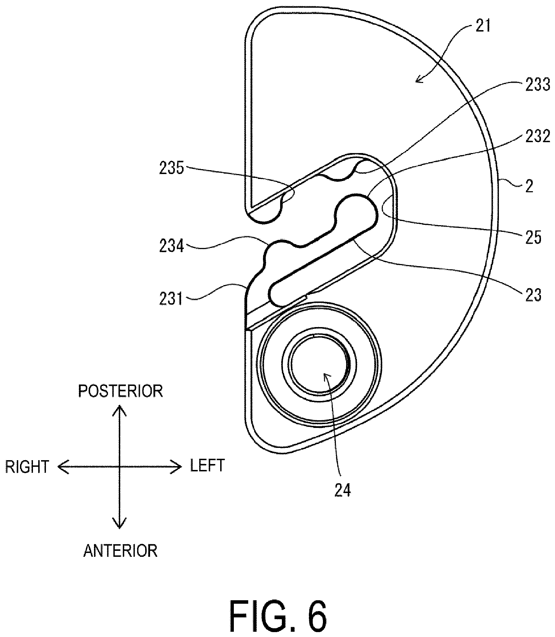

Next, the configuration of the tibial block 2 will be described in detail with reference to FIG 6. FIG. 6 is a plan view of the tibial block 2 as viewed from below.

The tibial block 2 has an upper surface 22, which faces the lower surface 11 of the tibial tray 1, and a lower surface 21 on the opposite side to the upper surface 22.

The tibial block 2 illustrated in FIG. 6 has a cut-out portion 25 formed so as to be attachable to the tibial tray 1 having the keel portion 14. Specifically, the cut-out portion 25 is formed at a position corresponding to the position of the keel portion 14 of the tibial tray 1 when the tibial block 2 is placed on the lower surface 11 of the tibial tray 1. When the tibial block 2 is in the attachment position, the keel portion 14 abuts against the cut-out portion 25.

The contact portion 23 is formed in the cut-out portion 25. The contact portion 23 protrudes into the space surrounded by the cut-out portion 25. The contact portion 23 is included in the fixing mechanism for fixing the tibial block 2 and the keel portion 14 in the vicinity of the cut-out portion 25.

As illustrated in FIG. 6, a protruding portion 233 and a protruding portion 235 protrude into the space defined by the cut-out portion 25. The contact portion 23 may, for instance, be a cantilever beam integrally formed with the tibial block 2 as illustrated in FIG. 6, but it is not limited to being this. In this case, the contact portion 23 includes a beam having a protruding portion 232 and a protruding portion 234, and a support portion 231 that supports the beam. The contact portion 23 may further include a protruding portion 233 and a protruding portion 235. The protruding portion 233 opposes the protruding portion 232 and the protruding portion 235 opposes the protruding portion 234. Note that the protruding portion 233 and the protruding portion 232 may be at any positions as long as they can receive a force from each other via the groove 141. In other words, the protruding portion 233 and protruding portion 232 need not be in direct opposition to each other.

The tibial block 2 may be formed with a through hole 24 as illustrated in FIG. 6. On an inner wall of the through hole 24, a female thread is formed to allow a male thread disposed around a later-described positioning pin 241 to be screwed in.

### Fixing Mechanism

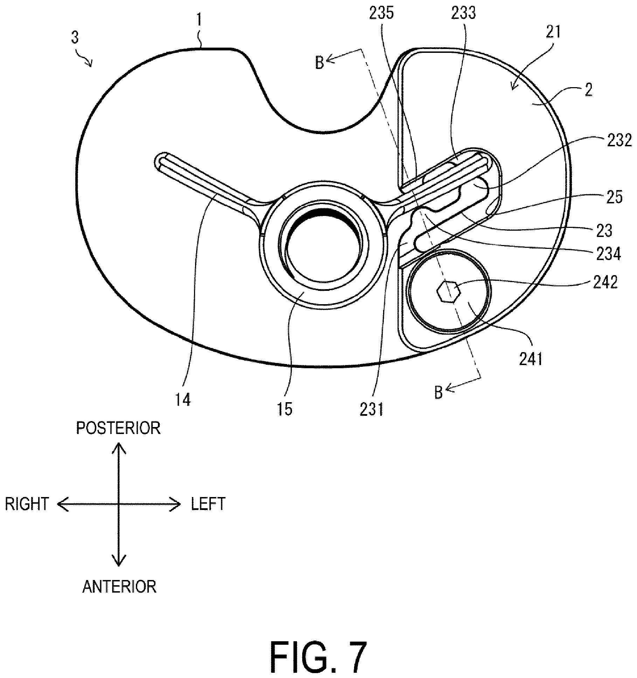

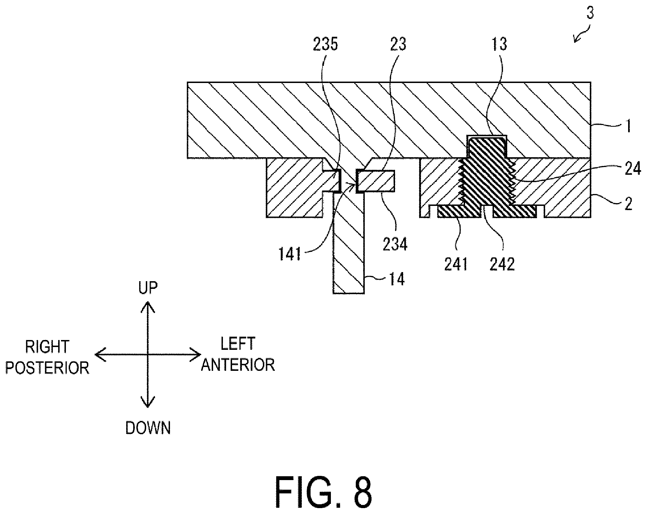

Next, the fixing mechanism for fixing the tibial block 2 to the tibial tray 1 is described with reference to FIGS. 7 and 8. FIG. 7 is a plan view of the tibial component 3 viewed from below. FIG. 8 is a cross-sectional view of the tibial component 3 taken along line B-B in FIG. 7.

When the tibial block 2 is mounted in the attachment position, as illustrated in FIGS. 7 and 8, the contact portion 23 is in contact with the groove 141 of the keel portion 14 so as to sandwich the keel portion 14 in the space defined by the cut-out portion 25.

As illustrated in FIG. 7, the keel portion 14 is sandwiched between the protruding portions 232 and 233, and between the protruding portions 234 and 235 in the groove 141. For example, when the tibial block 2 is placed in the attachment position, the protruding portions 232 and 233 are located in the portion between the protruding portions 142 and 143, and the protruding portions 234 and 235 are located in the portion between the protruding portion 143 and the stem portion 15 (see FIG. 5).

Note that, of the protruding portions 232 to 234, the protruding portion 232 may protrude much further than the other protruding portions, and a recessed portion to fit the protruding portion 232 may be provided at the bottom portion of the groove 141. In this case, the protruding portion 232 slides along the bottom portion of the groove 141 from the release position to the attachment position. While the protruding portion 232 slides in the groove 141, the contact portion 23 is deflected by a force in the left-right direction. When the tibial block 2 reaches the attachment position, the protruding portion 232 engages with the recessed portion provided in the groove 141. The protruding portion 232 engages with the recessed portion, thereby eliminating the deflection of the contact portion 23, fixing the tibial block 2 in the attachment position.

At least in part, when the tibial block 2 moves between the release position and the attachment position, the contact portion 23 applies force to the keel portion 14 in a direction substantially parallel to the lower surface 11 of the tibial tray 1. The beam supported by the support portion 231 of the contact portion 23 can be bent in the posterior direction (the left-anterior direction in FIG. 6). The contact portion 23 applies the force generated by the deflection of the beam to the keel portion 14. As a result, the tibial block 2 is fixed to the keel portion 14.

As illustrated in FIG. 6 and FIG. 7, the support portion 231 is provided on the entrance side of the cut-out portion 25 (which is to say the right-anterior direction in FIG. 6), while the contact portion 23 extends to the cut-out portion 25 (which is to say the left-posterior direction in FIG. 6). With this configuration, the deflection of the contact portion 23 can be used to easily attach the tibial block 2 to the tibial tray 1.

Note that while in the present specification a case in which the contact portion 23 is a cantilever beam has been described as an example, the shape of the contact portion 23 is not limited to this. The contact portion 23 may be any shape that is flexible and that can apply a force generated by deflection to the keel portion 14.

The tibial block 2, which is associated with the attachment position, may further be configured to be fixed using the positioning pin 241, as illustrated in FIG. 7.

As illustrated in FIGS. 7 and 8, the head of the positioning pin 241 has a groove 242 formed to fit a tool (such as a wrench) that can be used to rotate the positioning pin 241. A surgeon can then perform a procedure to rotate the positioning pin 241 inserted into the through hole 24 using the tool. FIG. 7 illustrates a positioning pin 241 having a hexagonal groove 242 formed therein.

When the tibial block 2 is in the attachment position, a tip end of the positioning pin 241 inserted from the lower surface 21 of the tibial block 2 reaches the recessed portion 13 through the through hole 24 as illustrated in FIG. 8. The tip end of the positioning pin 241 is accommodated in the recessed portion 13, thereby suppressing movement of the tibial block 2 from the attachment position.

### Modification Example

Note that, if the force of the contact portion 23 applied against the keel portion 14 is large enough to secure the tibial block 2 to the tibial tray 1, the positioning pin 241 is not required. In other words, the fixing mechanism included in the tibial component 3 does not require the through hole 24, the positioning pin 241, or the recessed portion 13.

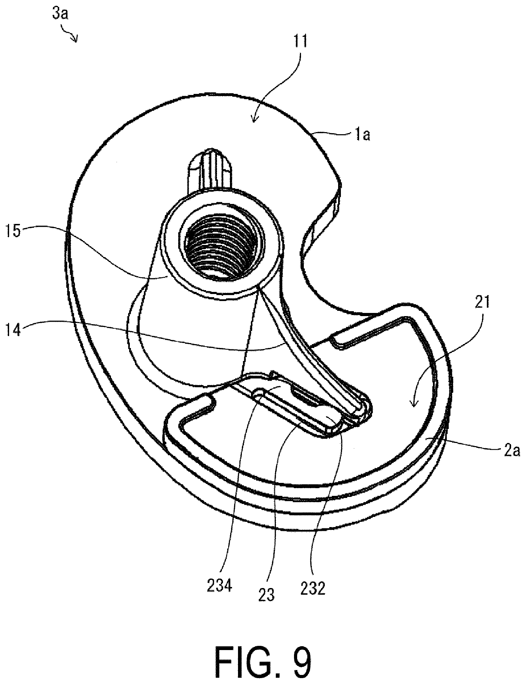

FIG. 9 is a perspective view of a tibial component 3a. FIG. 9 illustrates the tibial component 3a from below. A tibial block 2a, which has no through hole 24 or the positioning pin 241, is attached to a tibial tray 1a.

As illustrated in FIG. 9, in the tibial component 3a according to one aspect of the present invention, the tibial block 2a is fixed to the keel portion 14 disposed on the lower surface 11 of the tibial tray 1a.

### Second Embodiment

Next, another embodiment of the present invention will be described. Note that, for convenience of description, members having the same functions as those described in the above-described embodiments are denoted by the same reference numerals, and descriptions thereof will not be repeated.

### Configuration of Tibial Tray 1b

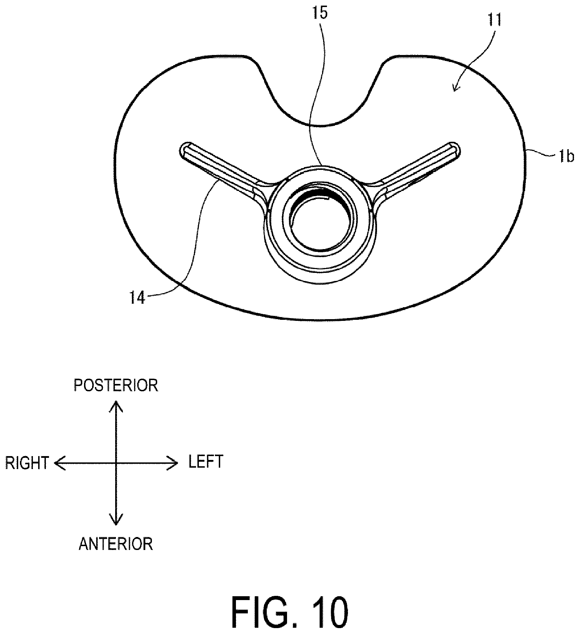

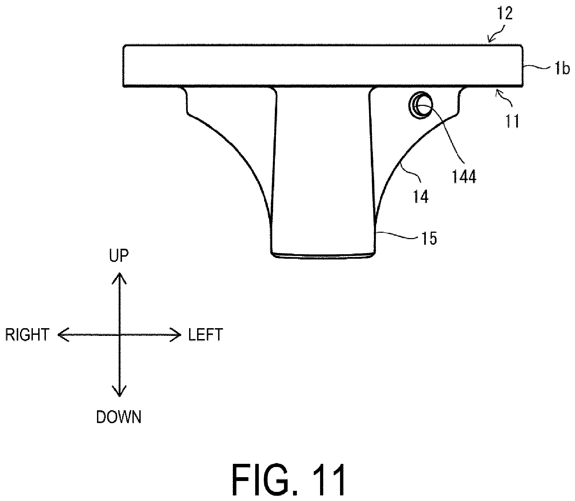

First, the configuration of the tibial tray 1b is described with reference to FIGS. 10 and 11. FIG. 10 is a plan view of the tibial tray 1b as viewed from below. FIG. 11 is a posterior view of the tibial tray 1b.

As illustrated in FIG. 10, there is no non-penetrating recessed portion 13 formed in the lower surface 11 (main surface) of the tibial tray 1b. As illustrated in FIG. 11, a first through hole 144 is formed in the keel portion 14 of the tibial tray 1b. A fixing pin 27 described below is inserted into the first through hole 144. In the tibial component 3b, this is the fixing mechanism that fixes the tibial block 2b to the keel portion 14 disposed on the lower surface 11 of the tibial tray 1b.

FIG. 11 illustrates a case where the tibial block 2b is to be positioned on the left side of the tibial tray 1b, but the configuration is not limited to this. In the tibial tray 1b, the first through hole 144 may also be formed in the right-side keel portion 14.

### Configuration of Tibial Block 2b

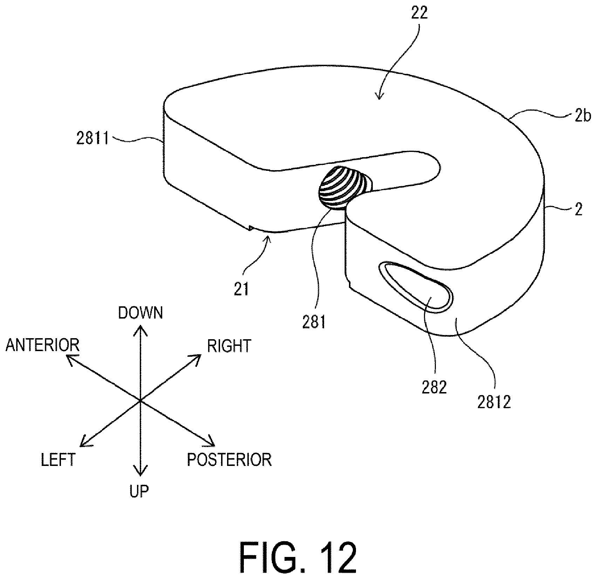

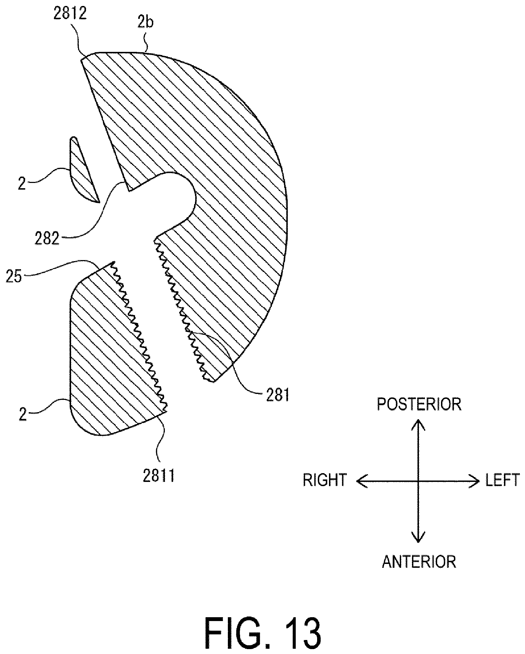

Next, the configuration of the tibial block 2b will be described with reference to FIGS. 12 and 13. FIG. 12 is a perspective view showing the tibial block 2b. FIG. 13 is a cross-sectional view of the tibial block 2b taken along a plane parallel to the top surface 22.

The tibial block 2b has an upper surface 22, which faces the lower surface 11 of the tibial tray 1b, and a lower surface 21, which is a surface on the opposite side to the upper surface 22. As illustrated in FIG. 13, in the tibial block 2b, a second through hole 281 is formed that reaches the cut-out portion 25 from a first peripheral edge portion 2811 of the tibial block 2b. Here, as illustrated in FIG. 12, the first peripheral edge portion 2811 is a portion on the anterior outer side of the tibial block 2b. Note that the fixing pin 27 may be inserted from the posterior side of the tibial block 2b.

As illustrated in FIG. 13, a female thread is formed in at least a portion of the inner periphery of the second through hole 281. Note that while FIGS. 12 and 13 show an example in which the female thread is formed throughout the inner periphery of the second through hole 281, the configuration is not limited to this. The only condition is that the portion of the outer periphery of the below-described fixing pin 27 on which the male thread is formed can be completely screwed into the second through hole 281. For example, the female thread may be formed only on the inner periphery of the first peripheral edge portion 2811 of the second through hole 281. Thus, the head of the fixing pin 27 does not protrude outward at the first peripheral edge portion 2811.

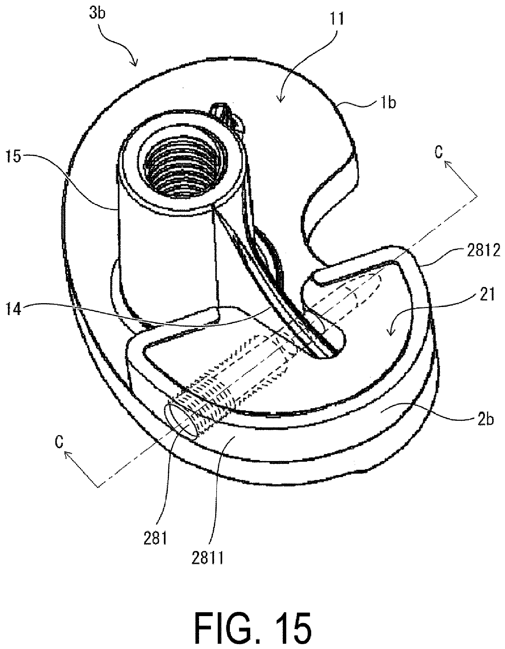

The second through hole 281 penetrates from the anterior side surface of the tibial block 2b to the side surface of the cut-out portion 25 in parallel with the upper surface 22 (see FIG. 15). When the tibial block 2b is mounted in the attachment position, the second through hole 281 faces the first through hole 144 formed in the cut-out portion 25.

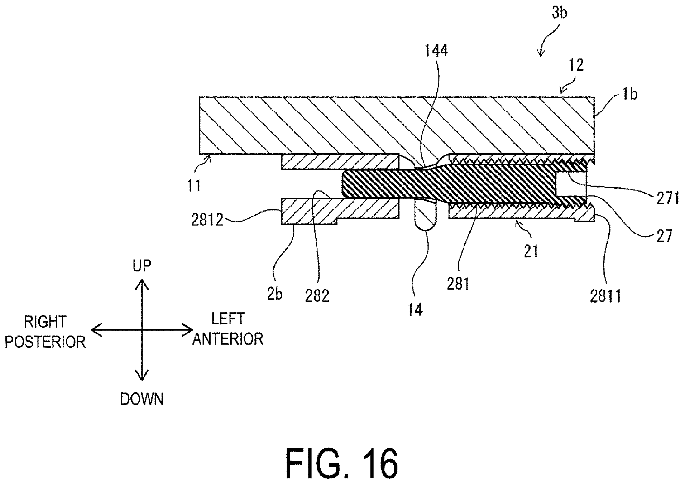

Further, as illustrated in FIG. 16, the tibial block 2b may further include a third through hole 282 that reaches the second peripheral edge portion 2812 of the tibial block 2b from the cut-out portion 25. Here, as illustrated in FIG. 12, the second peripheral edge portion 2812 is a portion on the posterior outer side of the tibial block 2b.

The third through hole 282 extends parallel to the upper surface 22 from the side surface of the cut-out portion 25 of the tibial block 2b to the side surface of the posterior side of the tibial block 2b (see FIG. 15). When the tibial block 2b is mounted in the attachment position, the third through hole 282 opposes the first through hole 144 formed in the keel portion 14. Further, the third through hole 282 is substantially parallel to the second through hole 281.

Note that instead of the third through hole 282, the tibial block 2b may be provided with a recessed portion (not illustrated) formed in the cut-out portion 25. The recessed portion is formed in a position so that, when the tibial block 2b is placed in the attachment position, it faces the first through hole 144 formed in the keel portion 14.

A tip end portion of the fixing pin 27 inserted into and through the second through hole 281 passes through the first through hole 144 and further reaches the recessed portion or the third through hole 282 facing the first through hole 144.

### Fixing Pin 27

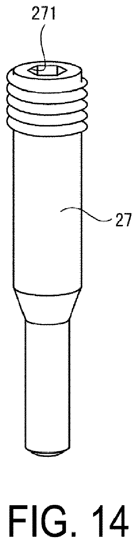

The fixing pin 27 is included in the fixing mechanism that fixes the tibial block 2 to the keel portion 14 disposed on the lower surface 11 of the tibial tray 1. FIG. 14 is a schematic view illustrating the fixing pin 27. As illustrated in FIG. 14, the fixing pin 27 is rod-shaped. At least a portion of the outer periphery of the fixing pin 27 has formed thereon a male thread that is screwed into the female thread formed on the inner periphery of the second through hole 281.

As illustrated in FIG. 14, the head of the fixing pin 27 has a groove 271 with an arbitrary shape suitable for a tool (such as, a wrench) that can be used to rotate the fixing pin 27. The surgeon can then perform a procedure to rotate the fixing pin 27 inserted into the second through hole 281 using the tool. FIG. 14 illustrates a fixing pin 27 having a hexagonal groove 271 formed therein.

The fixing pin 27 may have a rod shape with a diameter that is smaller at one end portion (the tip end side) than at the opposing end where the groove 271 is formed. In this case, the fixing pin 27 may be formed with a tapered portion where the diameter changes. In one example, when the fixing pin 27 is inserted into the first through hole 144, a tapered portion may be formed at a position of engagement with the first through hole 144.

### Supplementary Note

Note that in the tibial component 3, the lower surface 11 of the tibial tray 1 may have a left-right symmetric shape in plan view. When the tibial block 2 is disposed right-of-center or left-of-center of the lower surface 11, the tibial component 3 for the patient's left knee joint and the tibial component 3 for the patient's right knee joint can be common (in other words, members having the same shape can be used). Thus, the manufacturing costs of the tibial component 3 can be kept down.

Further, the upper surface 22 and the lower surface 21 of the tibial block 2 may have a reversible symmetric shape. In this case, the tibial block 2 may be fixed to the tibial tray 1 with the upper surface 22 contacting a first side that is either the right-of-center or the left-of-center side of the lower surface 11. Further, the tibial block 2 may be fixable to the tibial tray 1 with the lower surface 21 in contact with a second side that is opposite to the first side. With this configuration, either the upper surface 22 or the lower surface 21 of the tibial block 2 may be brought into contact with the right-of-center side or left-of-center side of the lower surface 11 of the tibial tray 1 to allow the tibial block 2 to be fixed to the tibial tray 1. Accordingly, the same tibial block 2 can be fitted on either the left or right side of the lower surface 11 of the tibial tray 1 and can be fitted face up or face down thereon. Thus, the manufacturing costs of the tibial component 3 can be kept down.

### Fixing Mechanism

Next, the fixing mechanism for fixing the tibial block 2b to the tibial tray 1b will described with reference to FIGS. 15 and 16. FIG. 15 is a perspective view of the tibial component 3b with the tibial block 2b located in the attachment position. FIG. 16 is a cross-sectional view of the tibial component 3b taken along the line C-C in FIG. 15.

The fixing mechanism applied to the tibial component 3b includes a first through hole 144 that penetrates the keel portion 14 and a second through hole 281 that reaches from the first peripheral edge portion 2811 to the cut-out portion 25 of the tibial block 2b, and a fixing pin 27. The fixing pin 27 is inserted through the second through hole 281 and reaches the first through hole 144.

As illustrated in FIGS. 15 and 16, the fixing pin 27 is inserted from the second through hole 281 in a direction substantially parallel to the lower surface 11. When the fixing pin 27 is inserted from the second through hole 281, the tip end of the fixing pin 27 reaches the space defined by the cut-out portion 25, and then reaches the first through hole 144 of the keel portion 14. Thus, the tibial block 2b and the keel portion 14 are fixed in the vicinity of the cut-out portion 25.

The tip end of the fixing pin 27 that has passed through the first through hole 144 may be configured to further reach a recessed portion (not illustrated) formed in the cut-out portion 25 or the third through hole 282. For example, as illustrated in FIG. 16, the fixing pin 27 inserted from the second through hole 281 may pass through the second through hole 281 and the first through hole 144, so that the tip end of the fixing pin 27 reaches the third through hole 282.

As illustrated in FIG. 16, in the tibial component 3b, the first through hole 144 may be tapered so that the opening diameter for the fixing pin 27 on the insertable side (the anterior side in FIG. 15 and the right side in FIG. 16) is greater than the opening diameter on the opposite side (the posteriors side in FIG. 15 and the left side in FIG. 16). At the site where the fixing pin 27 engages with the first through hole 144, the fixing pin 27 may have a tapered shape with a small diameter to the tip end side so as to allow tapered engagement with the first through hole 144. With this configuration, the tapered engagement between the fixing pin 27 and the first through hole 144 allows the tibial block 2b to be securely fixed to the tibial tray 2.

The present invention is not limited to the embodiments described above, and various modifications can be made within the scope of the claims, and further embodiments obtained by appropriately combining technical means disclosed in different embodiments are included in the technical scope of the present invention.

### Conclusion

(1) The tibial component according to one aspect of the present invention is a tibial component that forms a knee prosthesis, the tibial component including a tray having a main surface, a columnar portion disposed on the main surface, and a protruding portion protruding outward from the side surface of the columnar portion; an augment member disposed on the main surface; and a fixing mechanism configured to fix the augment member to the protruding portion.
   According to the configuration described above, the augment member is fixed to the protruding portion protruding outward from the side surface of the columnar portion disposed on the main surface of the tray. This allows the augment member to be attached to the tray using a simple procedure without forming a through hole in the tray.
(2) In the tibial component, the augment member may have a cut-out portion formed therein at a position corresponding to the position of the protruding portion when the augment member is placed in an attachment position on the main surface, and the fixing mechanism may fix the augment member and the protruding portion in a vicinity of the cut-out portion.
   The augment member disposed in the attachment position and the protruding portion are near each other at the cut-out portion. According to the configuration described above, the augment member and the protruding portion can be fixed in the vicinity of the cut-out portion, thereby attaching the augment member to the tray.
(3) In the tibial component, the fixing mechanism may include a contact portion formed in the cut-out portion, the contact portion being in contact with the protruding portion so as to sandwich the protruding portion.
(4) In the tibial component, the fixing mechanism may include at least one sliding groove formed in the protruding portion, the sliding groove having a bottom surface where the contact portion slides when the augment member is moved between the attachment position and a release position separated from the attachment position.
(5) In the tibial component, the contact portion applies a force in a direction substantially parallel to the main surface against the protruding portion at least in part when the augment member moves between the release position and the attachment position.
   In the configuration described above, the force of the contact portion applied against the protruding portion can be used to fix the augment member to the protruding portion and prevent the augment member from being separated from the protruding portion.
(6) In the tibial component, the contact portion may be a cantilever beam integrally formed with the augment member.
(7) In the tibial component, the augment member may be disposed left-of-center or right-of-center on the main surface.
(8) In the tibial component, the fixing mechanism may include a first through hole that penetrates the protruding portion, a second through hole that reaches the cut-out portion from a first peripheral edge portion of the augment member, and a fixing pin that is inserted into the second through hole and reaches the first through hole, and when the augment member is located at the attachment position, the fixing pin inserted from the second through hole may reach the first through hole.
(9) In the tibial component, a female thread may be formed on an inner periphery of the second through hole, and a male thread that engages with the female thread may be formed on at least a portion of an outer periphery of the fixing pin.
   According to the configuration described above, the augment member can be securely fixed to the protruding portion by screwing in the fixing pin.
(10) In the tibial component, in the fixing mechanism, the fixing pin may be inserted from the second through hole in a direction that is substantially parallel to the main surface.
(11) In the tibial component, the fixing mechanism further includes a recessed portion formed in the cut-out portion, or a third through hole that reaches a second peripheral edge portion of the augment member from the cut-out portion, and a tip end portion of the fixing pin inserted through the second through hole and passing through the first through hole and the second through hole reaches the recessed portion or the third through hole.
(12) In the tibial component, the main surface may have a left-right symmetric shape when seen in plan view, and the augment member may be disposed left-of-center or right-of-center.
   According to the configuration described above, the tibial component for the knee joint of the patient's right leg and the tibial component for the knee joint of the patient's left leg can be made using a common member, and the manufacturing cost of the tibial component can thus be kept down.
(13) In the tibial component, the augment member may have a first surface and a second surface on the opposite side to the first surface, and the first surface and second surface may have a reversible symmetric shape.
(14) In the tibial component, the augment member may be fixable to the tray with the first surface in contact with a first side that is one of a right-of-center side or a left-of-center side of the main surface, and may be fixable to the tray with the second surface being in contact with a second side that is on the opposite side to the first side.
   According to the configuration described above, the same augment member can be attached to either of the left and right sides of the main surface with either top-side up or top-side down, and thus the manufacturing costs of the members of the tibial component (such as the augment member) can be kept down.
(15) In the tibial component, the first through hole may have a tapered shape in which an opening diameter on a side where the fixing pin can be inserted is larger than an opening diameter on an opposite side, and the fixing pin may be tapered to have a smaller diameter at a tip end side, and, at a site of engagement with the first through hole, the fixing pin may allow tapered engagement with the first through hole.

According to the configuration described above, the augment member can be securely fixed to the tray by tapered engagement between the fixing pin and the first through hole.

### Reference Signs List

1, 1a, 1b Tibial tray (tray)
2, 2a, 2b Tibial block (augment member)
3, 3a, 3b Tibial component
11 Lower surface (main surface)
12 Upper surface
14 Keel portion (protruding portion)
15 Stem portion (columnar portion)
23 Contact portion
25 Cut-out portion
27 Fixing pin
100 Knee prosthesis
141 Groove (sliding groove)
144 First through hole
281 Second through hole
282 Third through hole
2811 First peripheral edge portion
2812 Second peripheral edge portion

## Claims

1. A tibial component that forms a knee prosthesis, the tibial component comprising:
a tray having a main surface, a columnar portion disposed on the main surface, and a protruding portion protruding outward from a side surface of the columnar portion;
an augment member disposed on the main surface; and
a fixing mechanism configured to fix the augment member to the protruding portion.

2. The tibial component according to claim 1, wherein
the augment member has formed therein a cut-out portion at a position corresponding to the position of the protruding portion when the augment member is placed at an attachment position on the main surface, and
the fixing mechanism fixes the augment member and the protruding portion in a vicinity of the cut-out portion.

3. The tibial component according to claim 2, wherein
the fixing mechanism comprises a contact portion formed in the cut-out portion, the contact portion being in contact with the protruding portion so as to sandwich the protruding portion.

4. The tibial component according to claim 3, wherein
the fixing mechanism comprises at least one sliding groove formed in the protruding portion, the sliding groove having a bottom surface where the contact portion slides when the augment member is moved between the attachment position and a release position separated from the attachment position.

5. The tibial component according to claim 4, wherein,
the contact portion applies a force in a direction substantially parallel to the main surface against the protruding portion at least in part when the augment member moves between the release position and the attachment position.

6. The tibial component according to claim 5, wherein
the contact portion is a cantilever beam integrally formed with the augment member.

7. The tibial component according to any one of claims 1 to 6, wherein
the augment member is disposed left-of-center or right-of-center on the main surface.

8. The tibial component according to claim 2, wherein
the fixing mechanism comprises a first through hole that penetrates the protruding portion, a second through hole that reaches the cut-out portion from a first peripheral edge portion of the augment member, and a fixing pin that is inserted into the second through hole and reaches the first through hole, and
when the augment member is located at the attachment position, the fixing pin inserted from the second through hole reaches the first through hole.

9. The tibial component according to claim 8, wherein
a female thread is formed on an inner periphery of the second through hole, and a male thread that engages with the female thread is formed on at least a portion of an outer periphery of the fixing pin.

10. The tibial component according to claim 8 or 9, wherein
in the fixing mechanism, the fixing pin is inserted from the second through hole in a direction that is substantially parallel to the main surface.

11. The tibial component according to any one of claims 8 to 10, wherein
the fixing mechanism further comprises a recessed portion formed in the cut-out portion, or a third through hole that reaches a second peripheral edge portion of the augment member from the cut-out portion, and
a tip end portion of the fixing pin inserted through the second through hole and passing through the first through hole and the second through hole reaches the recessed portion or the third through hole.

12. The tibial component according to any one of claims 8 to 11, wherein
the main surface has a left-right symmetric shape when seen in plan view, and the augment member is disposed left-of-center or right-of-center.

13. The tibial component according to any one of claims 8 to 12, wherein
the augment member has a first surface and a second surface on the opposite side to the first surface, and the first surface and second surface have a reversible symmetric shape.

14. The tibial component according to claim 13, wherein
the augment member is fixable to the tray with the first surface contacting one of a right-of-center side or a left-of-center side of the main surface, and is fixable to the tray with the second surface being in contact with the second side that is on the opposite side to the first side..

15. The tibial component according to any one of claims 11 to 14, wherein
the first through hole has a tapered shape in which an opening diameter on a side where the fixing pin can be inserted is larger than an opening diameter on an opposite side, and the fixing pin is tapered to have a smaller diameter at a tip end side and, at a position of engagement with the first through hole, the fixing pin allows tapered engagement with the first through hole.
